(19) Europäisches Patentamt
European Patent Office
Office européen des brevets

(11) EP 0 850 642 B1

(12) FASCICULE DE BREVET EUROPEEN

(45) Date de publication et mention de la délivrance du brevet:
**16.07.2003 Bulletin 2003/29**

(51) Int Cl.$^7$: **A61K 7/48**, A61K 7/06

(21) Numéro de dépôt: **97403010.8**

(22) Date de dépôt: **11.12.1997**

(54) **Composition comprenant une protéine d'origine végétale et/ou animale et un poly(acide 2-acrylamido 2-méthylpropane sulfonique) réticulé**

Zusammensetzung enthaltend ein Protein pflanzlicher und/oder tierischer Abkunft und eine vernetzte Poly(2-Acrylamido 2-Methylpropan Sulfonsäure)

Composition comprising a protein of plant and/or animal origin and a crosslinked poly(2-acrylamido 2-methylpropane sulfonic acid)

(84) Etats contractants désignés:
**DE ES FR GB IT**

(30) Priorité: **27.12.1996 FR 9616132**

(43) Date de publication de la demande:
**01.07.1998 Bulletin 1998/27**

(73) Titulaire: **L'OREAL**
**75008 Paris (FR)**

(72) Inventeur: **Lorant, Raluca**
**94320 Thiais (FR)**

(74) Mandataire: **Dodin, Catherine**
**L'OREAL-DPI**
**6 rue Bertrand Sincholle**
**92585 Clichy Cédex (FR)**

(56) Documents cités:
**FR-A- 2 698 004**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 850 642 B1

## Description

L'invention se rapporte à une composition cosmétique et/ou dermatologique contenant au moins une protéine d'origine végétale et/ou une protéine d'origine animale et au moins un polymère poly(acide 2-acrylamido 2-méthylpropane sulfonique) réticulé et neutralisé à au moins 90% ainsi qu'à ses utilisations.

Les compositions cosmétiques ou dermatologiques présentent en général une viscosité élevée et sont pour la plupart formulées sous une forme liquide épaissie telle qu'un lait, une crème, un gel ou une pâte. Ce type de présentation est très appréciée par le consommateur ; il correspond le plus souvent à une préoccupation pratique pour le formulateur : faciliter la prise du produit hors de son conditionnement sans perte significative, limiter la diffusion du produit à la zone locale de traitement et pouvoir l'utiliser dans des quantités suffisantes pour obtenir l'effet cosmétique ou dermatologique recherché.

Cet objectif est important pour les formulations telles que celles des produits pour le soin, l'hygiène ou le maquillage qui doivent bien s'étaler de façon homogène sur la surface locale à traiter.

Pour satisfaire à ces conditions, on augmente la viscosité des compositions par l'ajout de polymères épaississants et/ou gélifiants.

Au cours de ces dernières années, les macromolécules protéiques telles que les protéines de graine végétale : de céréale ou d'oléagineux et les protéines animales telles que les protéines lactiques sont très recherchées en cosmétique pour leurs propriétés hydratantes, leur bonne tolérance vis-à-vis de la peau et du corps humain.

L'introduction des protéines végétales dans les formulations cosmétiques provoque très souvent la déstabilisation de ces dernières qui se traduit le plus souvent par une chute importante de viscosité d'autant plus significative que le taux en protéine est élevé. De plus, à forte concentration, elles apportent un toucher collant peu cosmétique. Dans le cas des émulsions, les phénomènes d'instabilité peuvent se concrétiser aussi par des phénomènes de crémage, de coalescence ou de déphasage. Cette instabilité pourrait être due au mûrissement d'Ostwald. Pour stabiliser des formulations cosmétiques il faut utiliser des agents épaississants.

Les épaississants et/ou gélifiants lipophiles tels que les acides ou alcools gras sont en général efficaces mais ont tendance à apporter un toucher cosmétique gras et poisseux.

On préfère utiliser les épaississants et/ou les gélifiants hydrophiles qui apportent en général de la fraîcheur. Malheureusement, de nombreux agents épaississants et/ou gélifiants hydrophiles sont peu performants voire incompatibles avec les protéines végétales dont la présence entraîne une perte importante de leur pouvoir épaississant d'autant plus significative que le taux en protéine est élevé. D'autre part, l'augmentation de la concentration en polymère épaississant, en vue de stabiliser la formulation à base de protéine végétale, conduit le plus souvent à des effets indésirables sur le plan cosmétique tels qu'un effet collant et poisseux ou un aspect peu esthétique.

Par exemple, les gommes naturelles telles que la gomme xanthane ou les carraghénanes en présence de protéines végétales ont tendance à rendre les compositions fluides et filantes. Les gels aqueux à base de dérivés de cellulose perdent en partie leur viscosité en présence de protéines végétales.

Certains gélifiants comme les polyacryliques réticulés du type CARBOPOL sont incompatibles et conduisent, en présence de protéines végétales, à des gels aqueux granuleux, hétérogènes et instables.

Des mélanges de gélifiants hydrophiles tels que ceux à base de gomme de gellan et d'hydroxyéthylcellulose sont incompatibles et conduisent également, en présence de protéines végétales, à des gels aqueux granuleux, hétérogènes et instables.

Certaines protéines animales posent également des problèmes de compatibilité avec certains agents épaississants ou gélifiants. C'est le cas des protéines sériques telles que celles issues du sérum de cheval en présence des gélifiants comme les polyacryliques réticulés du type CARBOPOL; les gels aqueux les contenant se cassent et deviennent granuleux.

Un des objectifs de la présente invention est de pouvoir réaliser une grande variété de formulations cosmétiques ou dermatologiques à base de protéines, stables dans une large gamme de viscosités et présentant de bonnes propriétés cosmétiques au niveau de l'aspect, de la prise du produit et du toucher à l'application.

D'autre part, les protéines végétales ou animales sont pour la plupart difficilement utilisables dans les émulsions huile-dans-eau ou eau-dans-huile. Elles ont tendance à les fluidifier et à les rendre instables. Une des solutions pour résoudre ce problème serait d'augmenter le taux de tensioactifs émulsionnants pour stabiliser l'interface huile/eau mais ces derniers posent des problèmes d'innocuité du fait qu'ils présentent en général un potentiel irritant vis-à-vis de la peau et notamment pour les peaux sensibles.

La présente invention a donc également pour objectif de réaliser des émulsions huile-dans-eau ou eau-dans-huile stables et homogènes pouvant contenir de forts taux en protéines et présentant de bonnes propriétés cosmétiques au niveau de l'aspect, de la prise du produit et du toucher à l'application.

On connaît par FR2698004, des compositions cosmétiques sous forme de dispersion aqueuse comprenant, dans un milieu aqueux, un mono ou diester d'acide gras de sucre ou d'alkylsucre, et un copolymère réticulé d'acrylamide et d'un monomère choisi parmi l'acrylate d'ammonium, l'acide acrylamidométhylpropanesulfonique et le chlorure de

méthacryloyloxyéthyltriéthylammonium.

La demanderesse a découvert de manière surprenante une nouvelle famille de polymères épaississants et/ou gélifiants permettant d'obtenir des formulations cosmétiques et dermatologiques épaissies stables, à base de protéines d'origine végétale ou animale et qui apportent de bonnes propriétés cosmétiques telles qu'un toucher doux, non collant et lisse.

En effet, ces polymères particuliers permettent de réaliser des produits cosmétiques et/ou dermatologiques homogènes pouvant atteindre des viscosités élevées et stables dans le temps à température ambiante ou à des températures plus élevées.

Ils permettent notamment de réaliser des gels ou des sérums riches en protéines et présentant une bonne stabilité.

Ils permettent enfin de réaliser de façon surprenante des émulsions huile-dans-eau ou eau-dans-huile stables et homogènes pouvant contenir des protéines dans des quantités importantes sans apporter d'effet collant au toucher.

La présence d'huile dans les émulsions de l'invention à base de protéines, épaissies et stabilisées par les polymères particuliers définis ci-dessus, permet d'atténuer les effets collants apportés par les protéines utilisées à des taux de concentration importants et d'obtenir des produits cosmétiques légers et agréables à l'utilisation.

Enfin, les épaississants de l'invention permettent d'obtenir des émulsions huile-dans-eau riches en protéines et stables, sans qu'il soit nécessaire d'utiliser un tensioactif émulsionnant.

Ainsi, l'invention a pour objet une composition cosmétique et/ou dermatologique contenant dans un milieu cosmétiquement acceptable au moins une protéine d'origine végétale et/ou une protéine d'origine animale, hydrolysée ou non et au moins un poly(acide 2-acrylamido 2-méthylpropane sulfonique) réticulé et neutralisé à au moins 90%.

Les polymères poly(acide 2-acrylamido 2-méthylpropane sulfonique) réticulés et pratiquement ou totalement neutralisés, conformes à l'invention, sont hydrosolubles ou gonflables dans l'eau. Ils sont en général caractérisés par le fait qu'ils comprennent, distribués de façon aléatoire :

a) de 90 à 99,9% en poids de motifs de formule générale (1) suivante :

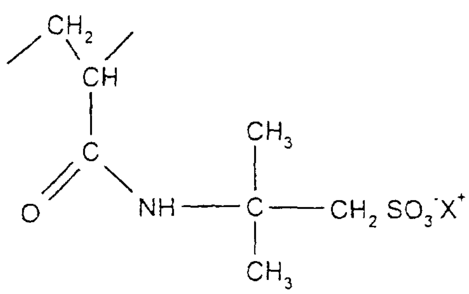

(1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles liaisons oléfiniques ; les proportions en poids étant définis par rapport au poids total du polymère.

De façon préférentielle, les polymères de l'invention comportent un nombre de motifs de formule (1) dans une quantité suffisamment élevée pour obtenir un volume hydrodynamique du polymère en solution d'eau ayant un rayon allant de 10 à 500 nm, de distribution homogène et unimodale.

Les polymères selon l'invention plus particulièrement préférés comprennent de 98 à 99,5% en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Les monomères de réticulation ayant au moins deux doubles liaisons oléfiniques sont choisis par exemple parmi le dipropylèneglycol-diallyléther, les polyglycoldiallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylène-bis-acrylamide ou le divinylbenzène.

Les monomères de réticulation ayant au moins deux doubles liaisons oléfiniques sont plus particulièrement choisis parmi ceux répondant à la formule générale (2) suivante :

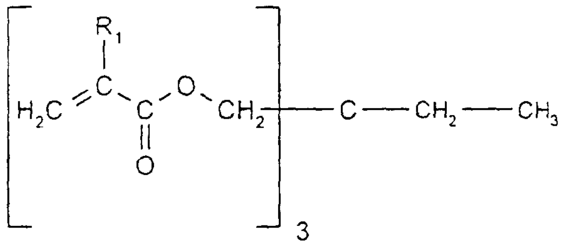 (2)

dans laquelle $R_1$ désigne un atome d'hydrogène ou un alkyle en $C_1$-$C_4$ et plus particulièrement méthyle (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par leur comportement rhéologique dans l'eau mais plus particulièrement par la diffusion de la lumière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec:

- $N_A$ désignant le nombre d'Avogadro ;
- $V_1$ désignant le volume spécifique du solvant ;
- $V_2$ désignant le volume spécifique de la macromolécule ;
- d désignant la masse en grammes du solvant qui est associé avec 1 gramme de macromolécule non dissoute
- M désignant la masse en grammes de la macromolécule non dissoute.

Si la particule hydrodynamique est sphérique, il est alors facile de calculer à partir du volume hydrodynamique le rayon hydrodynamique par la formule :

$$v_h = 4\Pi R^3/3$$

avec R désignant le rayon hydrodynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes polydispersés, on doit calculer la distribution des coefficients de diffusion. De cette distribution, on déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T est la température absolue en Kelvin, $\eta$ est la viscosité du solvant (eau) et R est

le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W.; Comp. Phys., 27, 213, 1982 ;
(5) Provincher S.W.; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al, Macromolecules, 1995, 28,4914-4919.

Les polymères particulièrement préférés sont ceux présentant une viscosité mesurée au viscosimètre BROOKFIELD dans une solution d'eau à 2% et à 25°C supérieure ou égale à 1000 cps et plus préférentiellement allant de 5000 à 40000 cps et plus particulièrement de 6500 à 35000 cps.

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol ;
(b) on neutralise la solution ou la dispersion de monomère AMPS obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 à 100% ;
(c) on ajoute à la solution ou dispersion obtenue en (b) le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Les poly(acide 2-acrylamido 2-méthylpropane sulfonique) réticulés, pratiquement ou totalement neutralisés sont présents dans les compositions cosmétiques ou dermatologiques de l'invention dans des concentrations allant préférentiellement de 0,01 à 20% en poids par rapport au poids total de la composition et plus préférentiellement de 0,1 à 10% en poids.

Les protéines utilisées selon l'invention sont celles couramment utilisées en cosmétique ou en dermatologie. Elles sont d'origine végétale ou animale, hydrolysées ou non hydrolysées.

Parmi les protéines végétales utilisables selon l'invention, on peut citer par exemple :

- les protéines de soja telles que le produit sous forme de dispersion aqueuse vendu sous le nom ELESERYL par la société LSN ;
- les protéines de blé tels que les hydrolysats vendus sous le nom TRITISOL par la société CRODA ou ceux vendus sous le nom VEGESERYL par la société LSN ;
- les protéines d'avoine telles que le produit REDUCTINE vendu par la société SILAB ;
- les protéines de pois telles que le produit ETIVAL vendu par COLETICA.

Parmi les protéines animales utilisables selon l'invention, on peut citer par exemple :

- les protéines de lait telles que la β-lactoglobuline, la caséine, le lactosérum;
- les protéines sériques telles que le sérum de cheval ;
- les protéines placentaires ;
- les protéines dermiques fibreuses telles que le collagène, l'élastine.

La ou les protéines sont présentes dans des concentrations allant de préférence de 0,001% à 30 % en poids, et plus préférentiellement de 0,01 % à 10 % en poids par rapport au poids total de la composition.

Les compositions de l'invention contiennent un milieu aqueux cosmétiquement acceptable, c'est-à-dire un milieu compatible avec toutes les matières kératiniques telles que la peau, les ongles, les muqueuses et les cheveux ou toute autre zone cutanée du corps.

Les compositions contiennent de préférence un milieu aqueux cosmétiquement et/ou dermatologiquement acceptable. Elles présentent un pH pouvant aller de préférence de 1 à 13 et plus préférentiellement de 2 à 12.

Le milieu cosmétiquement et/ou dermatologiquement acceptable des compositions selon l'invention est plus

particulièrement constitué d'eau et éventuellement de solvants organiques cosmétiquement et/ou dermatologiquement acceptables.

Les solvants organiques peuvent représenter de 5 % à 98 % du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des mono-alcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Afin que les compositions cosmétiques ou dermatologiques de l'invention soient plus agréables à utiliser (plus douce à l'application, plus nourrissante, plus émolliente), il est possible d'ajouter une phase grasse dans le milieu de ces compositions.

La phase grasse représente de préférence, de 0 % à 50 % du poids total de la composition.

Cette phase grasse peut comporter une ou plusieurs huiles choisies de préférence dans le groupe constitué par:

- les silicones volatiles ou non volatiles, linéaires, ramifiées ou cycliques, organo-modifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées,
- les esters d'acides gras.

Elle peut aussi comporter comme matière grasse un(e) ou plusieurs alcools gras, acides gras (acide stéarique) ou cires (paraffine, cires de polyéthylène, Carnauba, cire d'abeilles).

De façon connue, toutes les compositions de l'invention peuvent contenir des adjuvants habituels dans les domaines cosmétique et dermatologique, d'autres gélifiants et/ou épaississants classiques hydrophiles ou lipophiles ; des actifs hydrophiles ou lipophiles ; des conservateurs ; des antioxydants ; des parfums ; des émulsionnants ; des agents hydratants ; des agents pigmentants ; des dépigmentants ; des agents kératolytiques ; des vitamines ; des émollients ; des sequestrants ; des tensioactifs ; des polymères ; des agents alcanisants ou acidifiants ; des charges; des agents anti-radicaux libres ; des céramides ; des filtres solaires (notamment ultraviolets), des répulsifs pour insectes; des agents amincissants; des matières colorantes ; des bactéricides ; des antipelliculaires. Les quantités de ces différents adjuvants sont celles classiquement utilisées dans les domaines considérés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de solutions du type lotion ou sérum, sous forme de gels aqueux, sous forme d'émulsions obtenues par dispersion d'une phase grasse dans une phase aqueuse (H/E) ou inversement (E/H), de consistance liquide, semi-liquide ou solide telles que des laits, des crèmes plus ou moins onctueuses, des pâtes. Ces compositions sont préparées selon les méthodes usuelles.

Les compositions selon l'invention peuvent être utilisées comme produits rincés ou comme produits non-rincés capillaires notamment pour le lavage, le soin, le conditionnement, le maintien de la coiffure ou la mise en forme des fibres kératiniques telles que les cheveux.

Elles peuvent être des produits de coiffage tels que des lotions de mise en plis, des lotions pour le Brushing, des compositions de fixation et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple,

lorsqu'on souhaite obtenir un spray, une mousse pour la fixation ou le traitement des cheveux.
Les compositions de l'invention peuvent être également des shampooings, des compositions à rincer ou non, à appliquer avant ou après un shampooing, une coloration, une décoloration, une permanente ou un défrisage.
Les compositions de l'invention peuvent être également utilisées comme produit de soin et/ou d'hygiène tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.
Les compositions de l'invention peuvent être également utilisées comme produit antisolaire. Les compositions peuvent être des produits pour le maquillage.

Un autre objet de l'invention est un procédé de traitement non thérapeutique cosmétique de la peau, du cuir chevelu, des cheveux, des cils, des sourcils, des ongles ou des muqueuses, caractérisé par le fait qu'on applique sur le support kératinique une composition telle que définie ci-dessus, selon la technique d'utilisation habituelle de cette composition. Par exemple : application de crèmes, de gels, de sérums, de lotions, de laits sur la peau, le cuir chevelu et/ou les muqueuses. Le type de traitement est fonction de la ou des protéines présentes dans la composition.

L'invention a encore pour objet une utilisation de la composition ci-dessus pour préparer une lotion, un sérum, un lait, une pommade ou un onguent destiné à traiter thérapeutiquement les matières kératiniques telles que la peau, le cuir chevelu, les cheveux, les cils, les sourcils, les ongles ou les muqueuses.

Un autre objet de l'invention est l'utilisation d'un poly(acide 2-acrylamido 2-méthylpropane sulfonique) réticulé défini précédemment, comme agent stabilisant dans et pour la préparation d'une composition topique sous forme d'émulsion huile-dans-eau ou eau-dans-huile comportant des protéines d'origine végétale et/ou des protéines d'origine animale, hydrolysées ou non et plus particulièrement dans et pour la préparation d'une composition topique sous forme d'émulsion huile-dans-eau ne contenant pas de tensioactif.

Les exemples suivants illustrent l'invention sans présenter un caractère limitatif.

## EXEMPLE DE PREPARATION A

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minute dans une solution d'eau à 2 % et à 25°C allant de 15000 cps à 35000 cps. La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

## EXEMPLE DE PREPARATION B

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthylpropane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 19,2 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette

température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant une viscosité mesurée au viscosimètre BROOKFIELD, mobile 4, à une vitesse de rotation de 100 tours/minute dans une solution d'eau à 2 % et à 25°C de l'ordre de 7000 cps.

Le rayon hydrodynamique du polymère obtenu dans une solution aqueuse déterminé par diffusion de la lumière dynamique est de 160 nm.

**<u>EXEMPLE 1</u> : Gel de soin pour la peau**

- Poly(acide 2-acrylamido 2-méthylpropane sulfonique) réticulé et neutralisé par de l'ammoniaque, préparé selon le procédé de l'exemple A (viscosité de l'ordre de 16000 cps dans une solution d'eau à 2% et à 25°C) 2% en poids
- Protéines d'avoine (REDUCTINE de la société SILAB) 7,0 % en poids
- Conservateur qs
- Eau qsp 100 % en poids

<u>MODE OPERATOIRE :</u>

On disperse le polymère gélifiant dans l'eau et on homogénéise par une turbine du type Moritz jusqu'à l'obtention d'un gel lisse puis on ajoute les autres ingrédients dans l'ordre indiqué ci-dessous en maintenant l'agitation énergique.
On obtient un gel lisse, brillant, frais à l'application et laissant la peau douce et mate.

**<u>EXEMPLE 2</u> : Sérum sans émulsionnant pour le soin de la peau**

- Poly(acide 2-acrylamido 2-méthylpropane sulfonique) réticulé et neutralisé par de l'ammoniaque, préparé selon le procédé de l'exemple A (viscosité de l'ordre de 16.000 cps dans une solution d'eau à 2% et à 25°C) 0,8 % en poids
- Protéines de soja (ELESERYL de la société LSN) 20 % en poids
- Glycérol 5 % en poids
- Cyclométhicone 6 % en poids
- Conservateur qs
- Eau distillée qsp 100 % en poids

On opère dans les mêmes conditions que l'exemple 1.
On obtient un produit fluide, lisse ayant un toucher filmogène très doux.

**<u>EXEMPLE 3</u> : Crème de soin**

*Phase 1*

- Stéarate de polyéthylèneglycol à 20 moles d'OE (MYRJ49 par ICI) 1 % en poids
- Stéarate de glycéryle et stéarate de polyéthylèneglycol à 100 moles d'oxyde d'éthylène (ARLACEL165 par ICI) 1 % en poids
- acide stéarylique 2 % en poids
- alcool stéarique 1 % en poids
- isoparaffine hydrogénée 20 % en poids

*Phase 2*

- Poly(acide 2-acrylamido 2-méthylpropane sulfonique) réticulé et neutralisé par de l'ammoniaque, préparé selon le procédé de l'exemple de préparation A de viscosité de l'ordre de 16.000 cps dans une solution d'eau à 2% et à 25°C 1,5 % en poids
- Glycérol 3,0 % en poids
- Conservateur qs
- Eau qsp 100 % en poids

*Phase3*

- Hydrolysat de protéines de blé (TRITISOL XM de la société CRODA) 15 % en poids

**MODE OPERATOIRE :**

Les phases 1 et 2 sont homogénéisées et chauffées séparément à 70°C. On verse la phase huileuse 1 dans la phase aqueuse 2 sous une agitation énergique (turbine Moritz). Lorsque l'émulsion est fine, on refroidit le milieu sous agitation aux pales jusqu'à 35-40°C. On ajoute enfin la phase 3 protéique.
On obtient une crème épaisse, riche et nourrissante laissant la peau souple et lisse.

## Revendications

1. Composition cosmétique et/ou dermatologique, **caractérisée par le fait qu'**elle contient dans un milieu cosmétiquement acceptable au moins une protéine d'origine végétale et/ou une protéine d'origine animale, hydrolysée ou non et au moins un poly(acide 2-acrylamido 2-méthylpropane sulfonique) réticulé et neutralisé à au moins 90% qui comprend, distribués de façon aléatoire :

   a) de 90 à 99,9% en poids de motifs de formule générale (1) suivante :

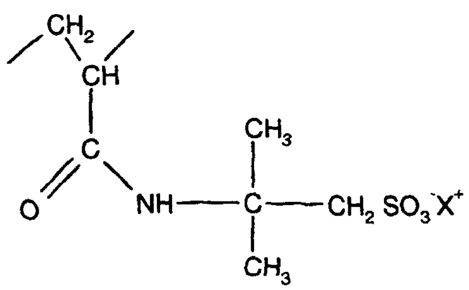

(1)

   dans laquelle $X^+$ désigne un cation ou un mélange de cations, au plus 10% mol des cations $X^+$ pouvant être des protons $H^+$ ;

   b) de 0,01 à 10% en poids de motifs réticulants provenant d'au moins un monomère ayant au moins deux doubles liaisons oléfiniques; les proportions en poids étant définis par rapport au poids total du polymère.

2. Composition selon la revendication 1, **caractérisée par le fait que** le poly(acide 2-acrylamido 2-méthylpropane sulfonique) réticulé comporte de 98 à 99,5% en poids de motifs de formule (1) et de 0,2 à 2% en poids de motifs réticulants.

3. Composition selon l'une quelconque des revendications 1 à 2, **caractérisée par le fait que** dans la formule (1) le cation $X^+$ est $NH_4^+$.

**4.** Composition selon l'une quelconque des revendications 1 à 3, **caractérisée par le fait que** les monomères réticulants répondent à la formule générale (2) suivante:

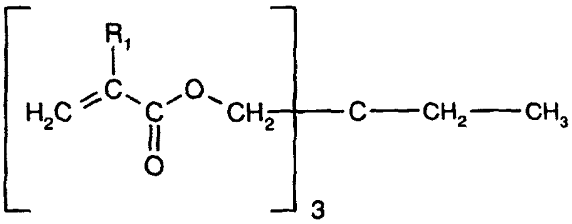

(2)

dans laquelle $R_1$ désigne un atome d'hydrogène ou un alkyle en $C_1$-$C_4$.

**5.** Composition selon l'une quelconque des revendications 1 à 4, **caractérisée par le fait que** le poly(acide 2-acrylamido 2-méthylpropane sulfonique) est réticulé par le triméthylol propane triacrylate.

**6.** Composition selon l'une quelconque des revendications 1 à 5, **caractérisée par le fait que** le poly(acide 2-acrylamido 2-méthylpropane sulfonique) réticulé présente une viscosité mesurée au viscosimètre BROOKFIELD, mobile 4, à une vitesse de rotation de 100 tours/minute, dans une solution d'eau à 2% et à 25°C supérieure ou égale à 1000 cps.

**7.** Composition selon l'une quelconque des revendications 1 à 6, **caractérisée par le fait que** le poly(acide 2-acrylamido 2-méthylpropane sulfonique) réticulé présente une viscosité mesurée au viscosimètre BROOKFIELD, mobile 4, à une vitesse de rotation de 100 tours/minute, dans une solution d'eau à 2% et à 25°C allant de 5000 à 40000 cps et plus particulièrement de 6500 à 35000 cps.

**8.** Composition selon l'une quelconque des revendications 1 à 7, **caractérisée par le fait que** le poly(acide 2-acrylamido 2-méthylpropane sulfonique) réticulé est présent dans des concentrations allant préférentiellement de 0,01 à 20% en poids par rapport au poids total de la composition et plus préférentiellement de 0,1 à 10% en poids.

**9.** Composition selon l'une quelconque des revendications 1 à 8, **caractérisée par le fait que** les protéines végétales sont choisies dans le groupe constitués par les protéines de soja ; les protéines de blé ; les protéines d'avoine ; les protéines de pois, ainsi que leurs hydrolysats.

**10.** Composition selon l'une quelconque des revendications 1 à 9, **caractérisée par le fait que** les protéines animales sont choisis dans le groupe constitué par les protéines de lait ; les protéines sériques ; les protéines placentaires ; les protéines dermiques fibreuses.

**11.** Composition selon l'une quelconque des revendications 1 à 10, **caractérisée par le fait que** les protéines sont présentes dans une concentration allant de 0,001% à 30% en poids, et de préférence de 0,01% à 10% en poids par rapport au poids total de la composition.

**12.** Composition selon l'une quelconque des revendications 1 à 11, **caractérisée en ce que** le milieu cosmétiquement et/ou dermatologiquement acceptable est constitué d'eau ou d'eau et d'au moins un solvant organique choisi dans le groupe constitué par les solvants organiques hydrophiles, les solvants organiques lipophiles, les solvants amphiphiles ou leurs mélanges.

**13.** Composition selon la revendication 12, **caractérisée en ce que** les solvants organiques sont choisis dans le groupe constitué par les alcools mono- ou polyfonctionnels, les polyéthylène glycols éventuellement oxyéthylénés, les esters de propylène glycol, le sorbitol et ses dérivés, les di-alkyles d'isosorbide, les éthers de glycol et les éthers de propylène glycol, les esters gras.

**14.** Composition selon la revendication 12 ou 13, **caractérisée en ce que** le ou les solvants organiques représentent de 5% à 98% du poids total de la composition.

**15.** Composition selon l'une quelconque des revendications 1 à 14, **caractérisée en ce qu'**elle comprend en plus au

moins une phase grasse.

**16.** Composition selon l'une quelconque des revendications 1 à 15, **caractérisée par le fait qu'**elle contient en plus au moins un additif choisi dans le groupe constitué par les gélifiants et/ou épaississants classiques hydrophiles ou lipophiles ; des actifs hydrophiles ou lipophiles ; des conservateurs ;des antioxydants ; des parfums ; des émulsionnants ; des agents hydratants ; des agents pigmentants ; des dépigmentants ; des agents kératolytiques ; des vitamines ; des émollients ; des séquestrants ; des tensioactifs ; des polymères ; des agents alcanisants ou acidifiants ; des charges ; des agents anti-radicaux libres ; des céramides ; des filtres solaires (notamment ultraviolets), des répulsifs pour insectes; des agents amincissants ; des matières colorantes ; des bactéricides ; des antipelliculaires.

**17.** Composition selon l'une quelconque des revendications 1 à 16, **caractérisée par le fait qu'**elle est utilisée comme produit capillaire rincé ou non-rincé pour le lavage, le soin, le conditionnement, le maintien de la coiffure ou la mise en forme des cheveux; comme produit de soin et/ou d'hygiène; comme produit de maquillage; comme produit antisolaire.

**18.** Procédé de traitement non thérapeutique et cosmétique de la peau, du cuir chevelu, des cheveux, des cils, des sourcils, des ongles ou des muqueuses, **caractérisé par le fait qu'**on applique sur le support une composition telle que définie selon l'une quelconque des revendications 1 à 17.

**19.** Utilisation de la composition selon l'une quelconque des revendications 1 à 17 pour préparer une lotion, un sérum, un lait, une pommade ou un onguent destiné à traiter thérapeutiquement la peau, le cuir chevelu, les cheveux, les cils, les sourcils, les ongles ou les muqueuses.

**20.** Utilisation d'un poly(acide 2-acrylamido 2-méthylpropane sulfonique) réticulé défini selon l'une quelconque des revendications 1 à 7, comme agent stabilisant dans et pour la préparation d'une composition topique sous forme d'émulsion huile-dans-eau ou eau-dans-huile comportant des protéines d'origine végétale et/ou des protéines d'origine animale, hydrolysées ou non.

**21.** Utilisation selon la revendication 20, dans laquelle la composition topique est sous forme d'émulsion huile-dans-eau sans tensioactif comportant une phase grasse et une phase aqueuse.

**Patentansprüche**

**1.** Kosmetische und/oder dermatologische Zusammensetzung, **dadurch gekennzeichnet, dass** sie in einem kosmetisch akzeptablen Medium mindestens ein Protein pflanzlichen Ursprungs und/oder ein Protein tierischen Ursprungs, das gegebenenfalls hydrolysiert ist, und mindestens eine vernetzte und zu mindestens 90 % neutralisierte Poly(2-acrylamido-2-methylpropansulfonsäure) enthält, die in zufälliger Verteilung enthält:

a) 90 bis 99,9 Gew.-% Einheiten der folgenden allgemeinen Formel (1):

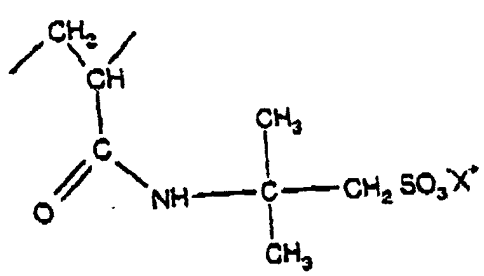

(1),

worin $X^+$ ein Kation oder ein Gemisch von Kationen bedeutet, wobei höchstens 10 Mol-% der Kationen $X^+$ Protonen $H^+$ sein können, und

b) 0,01 bis 10 Gew.-% vernetzende Einheiten, die von mindestens einem Monomer stammen, das mindestens zwei olefinische Doppelbindungen aufweist,

wobei die Gewichtsanteile bezogen auf das Gesamtgewicht des Polymers angegeben sind.

**2.** Zusammensetzung nach Anspruch 1, **dadurch gekennzeichnet, dass** die vernetzte Poly(2-acrylamido-2-methylpropansulfonsäure) 98 bis 99,5 Gew.-% Einheiten der Formel (1) und 0,2 bis 2 Gew.-% vernetzende Einheiten aufweist.

**3.** Zusammensetzung nach einem der Ansprüche 1 bis 2, **dadurch gekennzeichnet, dass** in der Formel (1) das Kation $X^+$ $NH_4^+$ ist.

**4.** Zusammensetzung nach einem der Ansprüche 1 bis 3, **dadurch gekennzeichnet, dass** die vemetzenden Monomere der folgenden allgemeinen Formel (2) entsprechen:

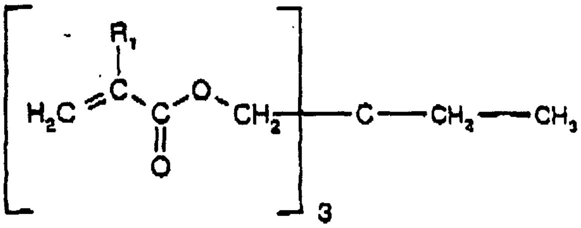

(2),

worin $R_1$ ein Wasserstoffatom oder eine $C_{1-4}$-Alkylgruppe bedeutet.

**5.** Zusammensetzung nach einem der Ansprüche 1 bis 4, **dadurch gekennzeichnet, dass** die Poly(2-acrylamido-2-methylpropansulfonsäure) mit Trimethylolpropantriacrylat vemetzt ist.

**6.** Zusammensetzung nach einem der Ansprüche 1 bis 5, **dadurch gekennzeichnet, dass** die vernetzte Poly(2-acrylamido-2-methylpropansulfonsäure) eine mit einem BROOKFIELD-Viskosimeter mit einem mobilen Teil 4 bei einer Rotationsgeschwindigkeit von 100 U/min in einer 2%igen wässerigen Lösung bei 25 °C bestimmte Viskosität von größer oder gleich 1 000 cP aufweist.

**7.** Zusammensetzung nach einem der Ansprüche 1 bis 6, **dadurch gekennzeichnet, dass** die vernetzte Poly(2-acrylamido-2-methylpropansulfonsäure) eine mit einem BROOKFIELD-Viskosimeter mit einem mobilen Teil 4 bei einer Rotationsgeschwindigkeit von 100 U/min in einer 2%igen wässerigen Lösung bei 25 °C bestimmte Viskosität von 5 000 bis 40 000 cP und insbesondere von 6 500 bis 35 000 cP aufweist.

**8.** Zusammensetzung nach einem der Ansprüche 1 bis 7, **dadurch gekennzeichnet, dass** die vernetzte Poly(2-acrylamido-2-methylpropansulfonsäure) in Konzentrationen vorliegt, die vorzugsweise im Bereich von 0,01 bis 20 Gew.-%, bezogen auf das Gesamtgewicht der Zusammensetzung, und noch bevorzugter im Bereich von 0,1 bis 10 Gew.-% liegen.

**9.** Zusammensetzung nach einem der Ansprüche 1 bis 8, **dadurch gekennzeichnet, dass** die pflanzlichen Proteine unter Sojaproteinen, Weizenproteinen, Haferproteinen, Erbsenproteinen sowie deren Hydrolysaten ausgewählt sind.

**10.** Zusammensetzung nach einem der Ansprüche 1 bis 9, **dadurch gekennzeichnet, dass** die tierischen Proteine unter Milchproteinen, Serumproteinen, Placentaproteinen und Faserproteinen der Haut ausgewählt sind.

**11.** Zusammensetzung nach einem der Ansprüche 1 bis 10, **dadurch gekennzeichnet, dass** die Proteine in einer Konzentration vorliegen, die im Bereich von 0,001 bis 30 Gew.-% und vorzugsweise im Bereich von 0,01 bis 10 Gew.-%, bezogen auf das Gesamtgewicht der Zusammensetzung, liegt.

**12.** Zusammensetzung nach einem der Ansprüche 1 bis 11, **dadurch gekennzeichnet, dass** das kosmetisch und/ oder dermatologisch akzeptable Medium aus Wasser oder aus Wasser und mindestens einem organischen Lösemittel besteht, das unter hydrophilen organischen Lösemitteln, lipophilen organischen Lösemitteln, amphiphilen Lösemitteln oder deren Gemischen ausgewählt ist.

**13.** Zusammensetzung nach Anspruch 12, **dadurch gekennzeichnet, dass** die organischen Lösemittel unter monofunktionellen oder polyfunktionellen Alkoholen, gegebenenfalls ethoxylierten Polyethylenglykolen, Propylenglykolestern, Sorbit und seinen Derivaten, Dialkylisosorbiden, Glykolethern, Propylenglykolethern und Fettestern ausgewählt sind.

**14.** Zusammensetzung nach Anspruch 12 oder 13, **dadurch gekennzeichnet, dass** das organische Lösemittel oder die organischen Lösemittel 5 % bis 98 % des Gesamtgewichts der Zusammensetzung ausmachen.

**15.** Zusammensetzung nach einem der Ansprüche 1 bis 14, **dadurch gekennzeichnet, dass** sie außerdem mindestens eine Fettphase enthält.

**16.** Zusammensetzung nach einem der Ansprüche 1 bis 15, **dadurch gekennzeichnet, dass** sie ferner mindestens einen Zusatzstoff enthält, der unter herkömmlichen hydrophilen oder lipophilen Gelbildnern und/oder Verdickungsmitteln, hydrophilen oder lipophilen Wirkstoffen, Konservierungsmitteln, Antioxidantien, Parfums, Emulgatoren, Hydratisierungsmitteln, pigmentierenden Mitteln, depigmentierenden Mitteln, Keratolytika, Vitaminen, Emollientien, Maskierungsmitteln, grenzflächenaktiven Stoffen, Polymeren, Mitteln zum Alkalischmachen oder zum Ansäuern, Füllstoffen, Radikalfängern für freie Radikale, Ceramiden, Sonnenschutzfiltern (insbesondere UV-Filtern), insektenabwehrenden Mitteln, schlankmachenden Mitteln, Farbmitteln, Bakteriziden oder Mitteln gegen Schuppen ausgewählt ist.

**17.** Zusammensetzung nach einem der Ansprüche 1 bis 16, **dadurch gekennzeichnet, dass** sie als Produkt zur Haarbehandlung, das ausgespült wird oder im Haar verbleibt, zum Waschen, zur Pflege, zum Konditionieren, für den Halt der Frisur oder für die Formgebung der Haare, als Pflegeprodukt und/oder Hygieneprodukt, als Schminkprodukt oder als Sonnenschutzprodukt verwendet wird.

**18.** Verfahren zur nichttherapeutischen und kosmetischen Behandlung der Haut, der Kopfhaut, der Haare, der Wimpern, der Augenbrauen, der Nägel oder der Schleimhäute, **dadurch gekennzeichnet, dass** eine in einem der Ansprüche 1 bis 17 definierte Zusammensetzung auf den Träger aufgebracht wird.

**19.** Verwendung der Zusammensetzung nach einem der Ansprüche 1 bis 17 zur Herstellung einer Lotion, eines Serums, einer Milch, einer Pomade oder einer Salbe, die zur therapeutischen Behandlung der Haut, der Kopfhaut, der Haare, der Wimpern, der Augenbrauen, der Nägel oder der Schleimhäute bestimmt ist.

**20.** Verwendung einer in einem der Ansprüche 1 bis 7 definierten vernetzten Poly(2-acrylamido-2-methylpropansulfonsäure) als Stabilisator in einer topischen Zusammensetzung und zur Herstellung einer topischen Zusammensetzung in Form einer Öl-in-Wasser-Emulsion oder einer Wasser-in-Öl-Emulsion, die Proteine pflanzlichen Ur-Sprungs und/oder Proteine tierischen Ursprungs enthält, die hydrolysiert oder nicht hydrolysiert vorliegen.

**21.** Verwendung nach Anspruch 20, worin die topische Zusammensetzung in Form einer Öl-in-Wasser-Emulsion ohne grenzflächenaktiven Stoff vorliegt, die eine Fettphase und eine wässerige Phase aufweist.

## Claims

**1.** cosmetic and/or dermatological composition, **characterized in that** it contains, in a cosmetically acceptable medium, at least one protein of plant origin and/or one protein of animal origin, which may or may not be hydrolysed, and at least one crosslinked poly(2-acrylamido-2-methylpropanesulphonic acid) neutralized to at least 90% which comprises, distributed randomly:

a) from 90 to 99.9% by weight of units of following general formula (1):

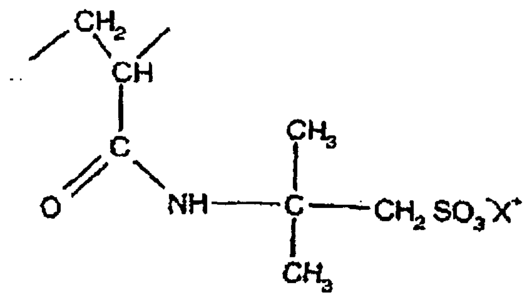

(1)

in which $X^+$ denotes a cation or a mixture of cations, it being possible for at most 10 mol% of the cations $X^+$ to be protons $H^+$;

b) from 0.01 to 10% by weight of crosslinking units resulting from at least one monomer having at least two olefinic double bonds, the proportions by weight being defined with respect to the total weight of the polymer.

2. Composition according to Claim 1, **characterized in that** the crosslinked poly(2-acrylamido-2-methylpropanesulphonic acid) contains from 98 to 99.5% by weight of units of formula (1) and from 0.2 to 2% by weight of crosslinking units.

3. Composition according to either one of Claims 1 and 2, **characterized in that**, in the formula (1), the cation $X^+$ is $NH_4^+$.

4. Composition according to any one of Claims 1 to 3, **characterized in that** the crosslinking monomers correspond to the following general formula (2):

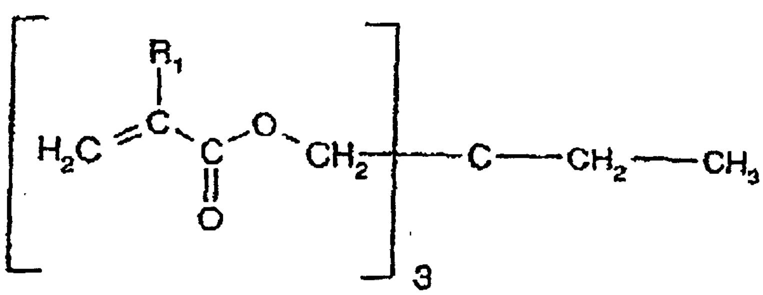

(2)

in which $R_1$ denotes a hydrogen atom or a $C_1$-$C_4$ alkyl.

5. Composition according to any one of Claims 1 to 4, **characterized in that** the poly(2-acrylamido-2-methylpropanesulphonic acid) is crosslinked with trimethylolpropane triacrylate.

6. Composition according to any one of Claims 1 to 5, **characterized in that** the crosslinked poly(2-acrylamido-2-methylpropanesulphonic acid) exhibits a viscosity, measured with a Brookfield viscometer, rotor 4, at a rotational speed of 100 revolutions/minute in a 2% solution in water at 25°C, of greater than or equal to 1000 cPs.

7. Composition according to any one of Claims 1 to 6, **characterized in that** the crosslinked poly(2-acrylamido-2-methylpropanesulphonic acid) exhibits a viscosity, measured with a Brookfield viscometer, rotor 4, at a rotational speed of 100 revolutions/minute in a 2% solution in water at 25°C, ranging from 5000 to 40,000 cPs and more particularly from 6500 to 35,000 cPs.

8. Composition according to any one of Claims 1 to 7, **characterized in that** the crosslinked poly(2-acrylamido-2-methylpropanesulphonic acid) is present in concentrations preferentially ranging from 0.01 to 20% by weight with respect to the total weight of the composition and more preferentially from 0.1 to 10% by weight.

9. Composition according to any one of Claims 1 to 8, **characterized in that** the plant proteins are chosen from the group composed of soya proteins; wheat proteins; oat proteins; pea proteins, and their hydrolysates.

10. Composition according to any one of Claims 1 to 9, **characterized in that** the animal proteins are chosen from the group composed of milk proteins; serum proteins; placental proteins; and fibrous skin proteins.

11. Composition according to any one of Claims 1 to 10, **characterized in that** the proteins are present in a concentration ranging from 0.001% to 30% by weight and preferably from 0.01% to 10% by weight with respect to the total weight of the composition.

12. Composition according to any one of Claims 1 to 11, **characterized in that** the cosmetically and/or dermatologically acceptable medium is composed of water or of water and of at least one organic solvent chosen from the group composed of hydrophilic organic solvents, lipophilic organic solvents, amphiphilic solvents or their mixtures.

13. Composition according to Claim 12, **characterized in that** the organic solvents are chosen from the group composed of mono- or polyfunctional alcohols, optionally oxyethylenated polyethylene glycols, propylene glycol esters, sorbitol and its derivatives, dialkyl isosorbides, glycol ethers and propylene glycol ethers, or fatty esters.

14. Composition according to Claim 12 or 13, **characterized in that** the organic solvent or solvents represent from 5% to 98% of the total weight of the composition.

15. Composition according to any one of Claims 1 to 14, **characterized in that** it additionally comprises at least one fatty phase.

16. Composition according to any one of Claims 1 to 15, **characterized in that** it additionally contains at least one additive chosen from the group composed of conventional hydrophilic or lipophilic gelling and/or thickening agents; hydrophilic or lipophilic active principles; preservatives; antioxidants; fragrances; emulsifiers; moisturizing agents; pigmenting agents; depigmenting agents; keratolytic agents; vitamins; emollients; sequestering agents; surfactants; polymers; basifying or acidifying agents; fillers; agents for combating free radicals; ceramides; sunscreen agents (in particular ultraviolet screening agents); insect repellents; slimming agents; colouring materials; bactericides; or antidandruff agents.

17. Composition according to any one of Claims 1 to 16, **characterized in that** it is used as rinse-out or leave-in hair product for washing, caring for, conditioning or form retention of the hairstyle or shaping the hair; as care and/or hygiene product; as make-up product; or as anti-sun product.

18. Process for the non-therapeutic and cosmetic treatment of the skin, scalp, hair, eyelashes, eyebrows, nails or mucous membranes, **characterized in that** a composition as defined according to any one of Claims 1 to 17 is applied on the substrate.

19. Use of the composition according to any one of Claims 1 to 17 for preparing a lotion, a serum, a milk, a pomade or an ointment intended for the therapeutic treatment of the skin, scalp, hair, eyelashes, eyebrows, nails or mucous membranes.

20. Use of a crosslinked poly(2-acrylamido-2-methylpropanesulphonic acid) defined according to any one of Claims 1 to 7 as stabilizing agent in and for the preparation of a topical composition in the form of an oil-in-water or water-in-oil emulsion containing proteins of plant origin and/or proteins of animal origin, which may or may not be hydrolysed.

21. Use according to Claim 20 in which the topical composition is in the form of a surfactant-free oil-in-water emulsion containing a fatty phase and an aqueous phase.